# EUROPÄISCHE PATENTSCHRIFT

(11) **EP 1 174 431 B1**
(45) Veröffentlichungstag und Bekanntmachung des Hinweises auf die Patenterteilung: **30.05.2012**
(21) Anmeldenummer: 01123321.0
(22) Anmeldetag: 31.10.1998
(51) Int. Cl.: C07D 487/04, A61K 31/53

(54) **2-Phenyl-substituierte Imidazotriazinone als Phoshodiesterase Inhibitoren**
2-Phenyl-substituited Imidazotriazinones as Phoshodiesterase Inhibitors
2-Phényl-substitués Imidazotriazinone comme inhibiteurs de la phoshodiesterase

(30) Priorität: 12.11.1997 DE 19750085; 23.03.1998 DE 19812462; 04.09.1998 DE 19840289
(43) Veröffentlichungstag der Anmeldung: 23.01.2002
(62) Teilanmeldung aus: 98959821.4
(73) Patentinhaber: Bayer Pharma Aktiengesellschaft, 13353 Berlin (DE)
(72) Erfinder: Niewöhner, Ulrich, Dr., 42929 Wermelskirchen (DE); Es-Sayed, Mazen, Dr., 40764 Langenfeld (DE); Haning, Helmut, Dr., Milford, CT 06460 (US); Schenke, Thomas, Dr., 51469 Bergisch Gladbach (DE); Schlemmer, Karl-Heinz, Dr., 42113 Wuppertal (DE); Keldenich, Jörg, Dr., 42113 Wuppertal (DE); Bischoff, Erwin, Dr., 42115 Wuppertal (DE); Perzborn, Elisabeth, Dr., 42327 Wuppertal (DE); Dembowsky, Klaus, Dr., Boston, MA 02116 (US); Serno, Peter, Dr., 51467 Bergisch Gladbach (DE); Nowakowski, Marc, Dr., 42115 Wuppertal (DE)

(56) Entgegenhaltungen:
- EP-A- 0 463 756
- WO-A-93/06104
- WO-A-93/07149
- WO-A-93/12095
- WO-A-94/00453
- WO-A-94/05661
- WO-A-94/28902
- WO-A-96/16657
- DE-A- 2 255 172
- DE-A- 2 364 076
- DE-A- 2 811 780
- CHARLES I ET AL: "BICYCLIC HETEROCYCLES WITH NITROGEN AT THE RING JUNCTION. PART 2.1 APPLICATION OF THE DAKIN-WEST REACTION TO THE SYNTHESIS OF IMIDAZO - 5,1-F-1,2,4-TRIAZIN-4(3H)-ONES" JOURNAL OF THE CHEMICAL SOCIETY, PERKIN TRANSACTIONS 1, Nr. 5, Mai 1980 (1980-05), Seiten 1139-1146, XP002027191
- DUMAITRE B ET AL: "SYNTHESIS AND CYCLIC GMP PHOSPHODIESTERASE INHIBITORY ACTIVITY OF ASERIES OF 6-PHENYLPYRAZOLOU3,4-DPYRIMIDONES" JOURNAL OF MEDICINAL CHEMISTRY, AMERICAN CHEMICAL SOCIETY. WASHINGTON, US, Bd. 39, Nr. 8, 1996, Seiten 1635-1644, XP002024856 Washington ISSN: 0022-2623
- RAJFER J ET AL: "NITRIC OXIDE AS A MEDIATOR OF RELAXATION OF THE CORPUS CAVERNOSUM IN RESPONSE TO NONADRENERGIC, NONCHOLINERGIC NEUROTRANSMISSION" NEW ENGLAND JOURNAL OF MEDICINE, THE, MASSACHUSETTS MEDICAL SOCIETY, WALTHAM, MA, US, Bd. 362, Nr. 2, 9. Januar 1992 (1992-01-09), Seiten 90-94, XP000943589 ISSN: 0028-4793
- COSTE H ET AL: "CHARACTERIZATION OF A NOVEL POTENT AND SPECIFIC INHIBITOR OR TYPE VPHOSPHODIESTERASE" BIOCHEMICAL PHARMACOLOGY, PERGAMON, OXFORD, GB, Bd. 50, Nr. 10, 1995, Seiten 1577-1585, XP000973413 ISSN: 0006-2952
- MURRAY K J: "THE THERAPEUTIC POTENTIAL OF PDE VA INHIBITORS AS, FOR EXAMPLE, VASODILATORS, BRONCHODILATORS AND MODULATORS OF GASTROINTESTINAL MOTILITY, IS LARGELY BASED ON THE EFFECTS OF ONE COMPOUND, ZAPRINAST, AND A CLEARER PICTURE WILL BE OBTAINED WHEN OTHER RATIONALLY DESIGNED INHIBITORS BECOME AVAILABLE" DRUG NEWS AND PERSPECTIVES, XX, XX, Bd. 6, Nr. 3, April 1993 (1993-04), Seiten 150-156, XP000943556 ISSN: 0214-0934

## Beschreibung

Die vorliegende Erfindung betrifft Verfahren zu Herstellung von 2-Phenyl-substituierte Imidazotriazione und Zwischenprodukte.

In der Offenlegungsschrift DE 28 11 780 sind Imidazotriazine als Bronchodilatoren mit spasmolytischer Aktivität und Hemmaktivität gegen cyclisches Adenosinmonophosphat metabolisierende Phosphodiesterasen (cAMP-PDE's, Nomenklatur nach Beavo: PDE-III und PDE-IV) beschrieben. Eine Hemmwirkung gegen cylisches Guanosin-monophosphat metabolisierende Phosphodiesterasen (cGMP-PDE's, Nomenklatur nach Beavo und Reifsnyder (Trends in Pharmacol. Sci. 11, 150-155, 1990) PDE-I, PDE-II und PDE-V) ist nicht beschrieben. Es werden keine Verbindungen beansprucht, die eine Sulfonamidgruppe im Arylrest in der 2-Position enthalten. Weiterhin werden Imidazotriazinone in FR 22 13 058, CH 59 46 71, DE 22 55 172, DE 23 64 076 und EP 000 9384 beschrieben, die in der 2-Position keinen substituierten Arylrest besitzen, und ebenfalls als Bronehodilatatoren mit cAMP-PDE inhibitorischer Wirkung beschrieben werden.

In WO 94/28902 werden Pyrazolopyrimidinone beschrieben, die sich für die Behandlung von Impotenz eignen.

Die erfindungsgemäßen Verbindungen sind potente Inhibitoren von entweder einer oder mehrerer der cyclisches Guanosin 3',5'-monophosphat metabolisierenden Phosphodiesterasen (cGMP-PDE's). Entsprechend der Nomenklatur von Beavo und Reifsnyder (Trends in Pharmacol. Sci. 11, 150-155, 1990) handelt es sich um die Phosphodiesterase Isoenzyme PDE-I, PDE-II und PDE-V.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Kugnier, Exp. Opin. Invest. Drugs (1995), 4 (11), 1081 -1100).

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di- bzw. Trimethylamin, Di- bzw. Triethanolamin, Dicyclohexylamin, Dimethylaminoethanol, Arginin, Lysin, Ethylendiamin oder 2-Phenylethylamin.

Heterocyclus, gegebenenfalls benzokondensiert, steht im Rahmen der Erfindung im allgemeinen für einen gesättigten, partiell ungesättigten oder ungesättigten 5- bis 7-gliedrigen Heterocyclus, der bis zu 4 Heteroatome aus der Reihe S, N und/oder O enthalten kann. Beispielsweise seien genannt: Azepin, Diazepin, Indolyl, Isochinolyl, Chinolyl, Benzo[b]thiophen, Benzo[b]furanyl, Pyridyl, Thienyl, Tetrahydrofuranyl, Tetrahydropyranyl, Furyl, Pyrrolyl, Thiazolyl, Triazolyl, Tetrazolyl, Isoxazolyl, Imidazolyl, Morpholinyl, Thiomorpholinyl, Pyrrolidinyl, Piperazinyl, N-Methylpiperazinyl oder Piperidinyl. Bevorzugt sind Chinolyl, Furyl, Pyridyl, Thienyl, Piperidinyl, Pyrrolidinyl, Piperazinyl, Azepin, Diazepin, Thiazolyl, Triazolyl, Tetrazolyl, Tetrahydrofuranyl, Tetrahydropyranyl, Morpholinyl und Thiomorpholinyl.

Ein geradkettiger oder verzweigter Acylrest mit 1 bis 6 Kohlenstoffatomen steht im Rahmen der Erfindung beispielsweise für Acetyl, Ethylcarbonyl, Propylcarbonyl, Isopropylcarbonyl, Butylcarbonyl, Isobutylcarbonyl, Pentylcarbonyl und Hexylcarbonyl. Bevorzugt ist ein geradkettiger oder verzweigter Acylrest mit 1 bis 4 Kohlenstoffatomen. Besonders bevorzugt sind Acetyl und Ethylcarbonyl.

Ein geradkettiger oder verzweigterAlkoxyrest mit 1 bis 6 bzw. 1 bis 4 Kohlenstoffatomen steht im Rahmen der Erfindung für Methoxy, Ethoxy, n-Propoxy, Isopropoxy, tert.Butoxy, n-Pentoxy und n-Hexoxy. Bevorzugt ist ein geradkettiger oder verzweigter Alkoxyrest mit 1 bis 6, 1 bis 4 bzw. 1 bis 3 Kohlenstoffatomen. Besonders bevorzugt ist ein geradkettiger oder verzweigter Alkoxyrest mit 1 bis 3 Kohlenstoffatomen.

Ein geradkettiger oder verzweigter Alkoxycarbonylrest mit 1 bis 6 Kohlenstoffatomen steht im Rahmen der Erfindung beispielsweise für Methoxycarbonyl, Ethoxycarbonyl, n-Propoxycarbonyl, Isopropoxycarbonyl und tert.Butoxycarbonyl. Bevorzugt ist ein geradkettiger oder verzweigter Alkoxycarbonylrest mit 1 bis 4 Kohlenstoffatomen. Besonders bevorzugt ist ein geradkettiger oder verzweigter Alkoxycarbonylrest mit 1 bis 3 Kohlenstoffatomen.

Ein geradkettiger oder verzweigter Alkylrest mit 1 bis 4, 1 bis 6, 1 bis 8 und 1 - 10 Kohlenstoffatomen steht im Rahmen der Erfindung beispielsweise für Methyl, Ethyl, n-Propyl, Isopropyl, tert.Butyl, n-Pentyl, n-Hexyl, n-Heptyl, n-Octyl, n-Nonyl und n-Decyl. Bevorzugt sind geradkettige oder verzweigte Alkylreste mit 1 bis 3, 1 bis 4 bzw. 1 bis 8 Kohlenstoffatomen. Besonders bevorzugt sind geradkettige oder verzweigte Alkylreste mit 1 bis 4 bzw. 1 bis 3 Kohlenstoffatomen.

Geradkettiges Alkyl mit bis zu 4 Kohlenstoffatomen steht im Rahmen der Erfindung beispielsweise für Methyl, Ethyl, n-Propyl und n-Butyl.

(C₆-C₁₀)-Aryl steht im allgemeinen für einen aromatischen Rest mit 6 bis 10 Kohlenstoffatomen. Bevorzugte Arylreste sind Phenyl und Naphthyl.

Cycloalkyl mit 3 bis 8 bzw. 3 bis 7 Kohlenstoffatomen steht im Rahmen der Erfindung beispielsweise für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl. Cycloalkyloxy mit 3 bis 8 Kohlenstoffatomen steht im Rahmen der Erfindung für Cyclopropyloxy, Cyclopentyloxy, Cyclobutyloxy, Cyclohexyloxy, Cycloheptyloxy oder Cyclooctyloxy. Bevorzugt seien genannt: Cyclopropyloxy, Cyclopentyloxy und Cyclohexyloxy.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Ein 5- bis 6-gliedriger bzw. 7-gliedriger gesättigter Heterocyclus, der ein weiteres Heteroatom aus der Reihe S, N und/oder O enthalten kann steht im Rahmen der Erfindung und in Abhängigkeit der oben aufgeführten Substituenten beispielsweise für Morpholinyl, Piperidinyl, Piperazinyl, Tetrahydropyranyl oder Tetrahydrofuranyl. Bevorzugt sind Morpholinyl, Tetrahydropyranyl, Piperidinyl und Piperazinyl.

Ein 5- bis 6-gliedriger aromatischer Heterocyclus mit bis zu 3 oder 4 Heteroatomen aus der Reihe S, O und/oder N steht im Rahmen der Erfindung beispielsweise für Pyridyl, Pyrimidyl, Pyridazinyl, Thienyl, Furyl, Pyrrolyl, Thiazolyl, Oxazolyl oder Imidazolyl. Bevorzugt sind Pyridyl, Pyrimidyl, Pyridazinyl, Furyl und Thiazolyl.

Ein 5- bis 6-gliedriger ungesättigter, partiell ungesättigter und gesättigter Heterocyclus, der bis zu 3 bzw. 4 Heteroatome aus der Reihe S, O und/oder N enthalten kann, steht im Rahmen der Erfindung beispielsweise für Pyridyl, Pyrimidyl, Pyridazinyl, Thienyl, Furyl, Pyrrolyl, Thiazolyl, Oxazolyl, Imidazolyl, Piperidinyl, Piperazinyl oder Morpholinyl. Bevorzugt sind Pyridyl, Pyrimidyl, Piperazinyl, Pyridazinyl, Morpholinyl, Furyl und Thiazolyl.

Die erfindungsgemäßen Verbindungen, insbesondere die Salze, können auch als Hydrate vorliegen. Im Rahmen der Erfindung werden unter Hydraten solche Verbindungen verstanden, die im Kristall Wasser enthalten. Solche Verbindungen können ein oder mehrere, typischerweise 1 bis 5, Äquivalente Wasser enthalten. Hydrate lassen sich beispielsweise herstellen, indem man die betreffende Verbindung aus Wasser oder einem wasserhaltigen Lösungsmittel kristallisiert.

Außerdem wurde ein Verfahren zur Herstellung der erfindungsgemäßen Verbindungen der allgemeinen Formel (I) gefunden, dadurch gekennzeichnet, daß man zunächst Verbindungen der allgemeinen Formel (II) 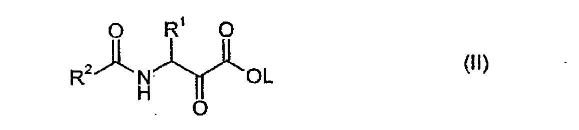 in welcher
- R¹, R²: für Propyl R² für Propyl stehan für Methyl
und
- L: für geradkettiges oder verzweigtes Alkyl mit bis zu 4 Kohlenstoffatomen steht,
mit Verbindungen der allgemeinen Formel (III) 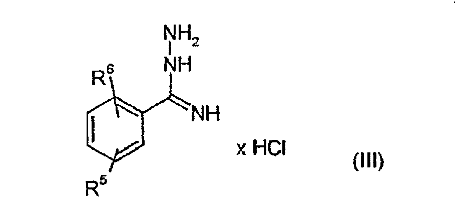 in welcher
R¹ für wasser staff und R⁶ für eine in 1-Position ständige Ethoxy gruppe steht,
in einer Zweistufenreaktion in den Systemen Ethanol und Phosphoroxytrichlorid / Dichlorethan in die Verbindungen der allgemeinen Formel (IV) 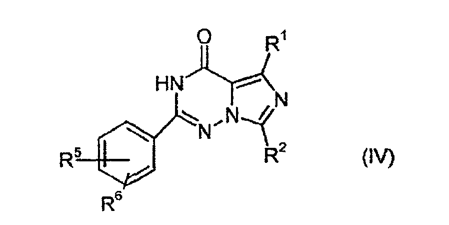 in welcher
R¹, R², R⁵ und R⁶ die oben angegebene Bedeutung haben,
überführt, in einem weiteren Schritt mit Chlorsulfonsäure zu den Verbindungen der allgemeinen Formel (V) 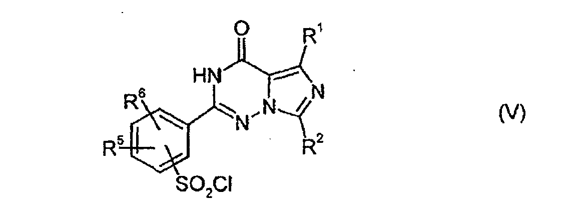 in welcher
R¹, R², R⁵ und R⁶ die oben angegebene Bedeutung haben,
umsetzt und abschließend mit Aminen der allgemeinen Formel (VI)

HN³R⁴ (VI)

in welcher
R³ und R⁴ die oben angegebene Bedeutung haben,
in inerten Lösemitteln umsetzt.

Das erfindungsgemäße Verfahren kann durch folgendes Formelschema beispielhaft erläutert werden:

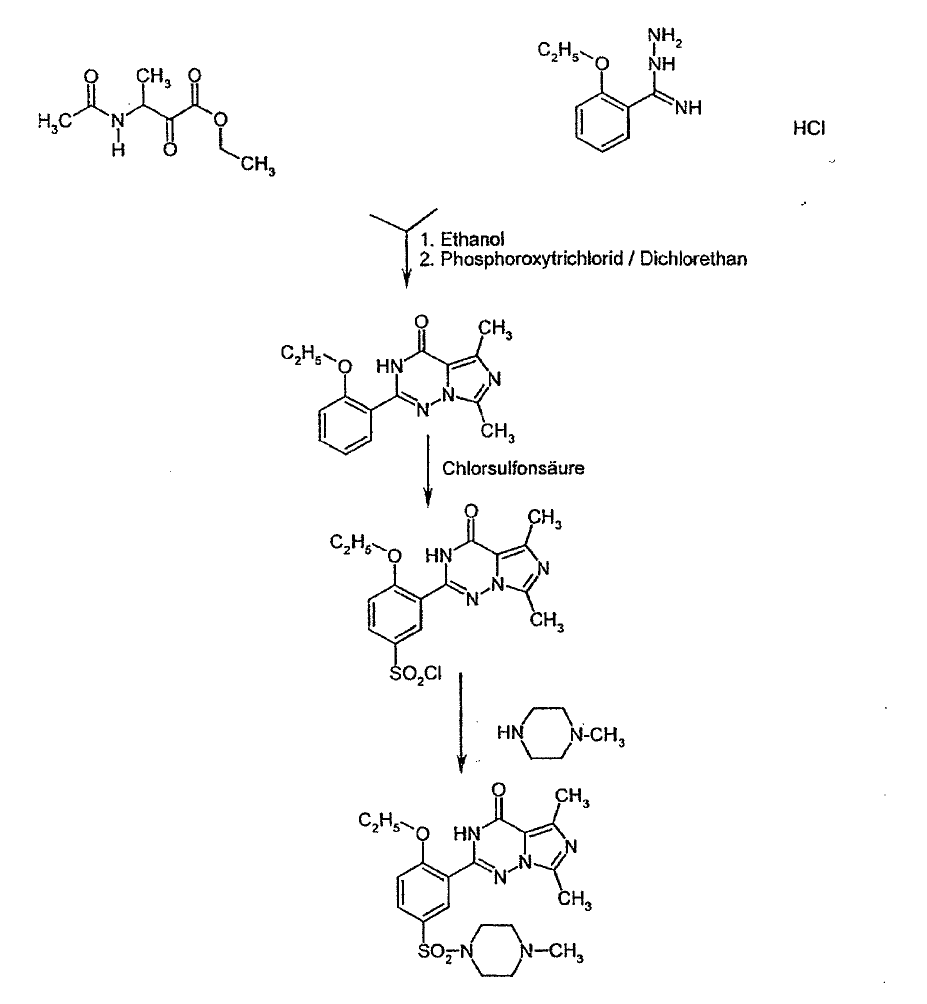

Als Lösemittel für die einzelnen Schritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Ethanol und für den zweiten Schritt Dichlorethan..

Die Reaktionstemperatur kann im allgemeinen in einem größeren Bereich variiert werden. Im allgemeinen arbeitet man in einem Bereich von -20°C bis 200°C, bevorzugt von 0°C bis 70°C.

Die erfindungsgemäßen Verfahrensschritte werden im allgemeinen bei Normaldruck durchgeführt. Es ist aber auch möglich, bei überdruck oder bei Unterdruck durchzuführen (z.B. in einem Bereich von 0,5 bis 5 bar).

Die Umsetzung zu den Verbindungen der allgemeinen Formel (V) erfolgt in einem Temperaturbereich von 0°C bis Raumtemperatur und Normaldruck.

Die Umsetzung mit den Aminen der allgemeinen Formel (VI) erfolgt in einem der oben aufgeführten chlorierten Kohlenwasserstoffe, vorzugsweise in Dichlormethan.

Die Reaktionstemperatur kann im allgemeinen in einem größeren Bereich variiert werden. Im allgemeinen arbeitet man in einem Bereich von -20°C bis 200°C, bevorzugt von 0°C bis Raumtemperatur.

Die Umsetzung wird im allgemeinen bei Normaldruck durchgeführt. Es ist aber auch möglich, bei Überdruck oder bei Unterdruck durchzuführen (z.B. in einem Bereich von 0,5 bis 5 bar).

Die Verbindungen der allgemeinen Formel (II) sind teilweise bekannt oder neu und können dann hergestellt werden, indem man

Verbindungen der allgemeinen Formel (VII)

R²-CO-T (VII)

in welcher
- R²: die oben angegebene Bedeutung hat
und
- T: für Halogen, vorzugsweise für Chlor steht,
zunächst durch Umsetzung mit Verbindungen der allgemeinen Formel (VIII) 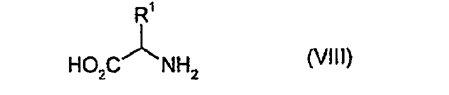 in welcher
- R¹: die oben angegebene Bedeutung hat
in inerten Lösemitteln, gegebenenfalls in Anwesenheit einer Base und Trimethylsilylchlorid in die Verbindungen der allgemeinen Formel (IX) 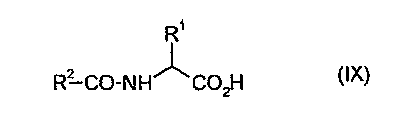 in welcher
R¹ und R² die oben angegebene Bedeutung haben,
überführt und abschließend mit der Verbindung der Formel (X) 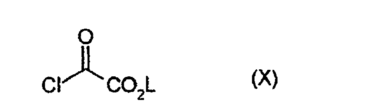 worin L die oben angegebene Bedeutung hat,
in inerten Lösemitteln, gegebenenfalls in Anwesenheit einer Base umsetzt.

Als Lösemittel für die einzelnen Schritte des Verfahrens eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlonnethan, Dichlorethylen, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösemittel zu verwenden. Besonders bevorzugt ist für den ersten Schritt Dichlormethan und für den zweiten Schritt ein Gemisch aus Tetrahydrofuran und Pyridin.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Pyridin und/oder Dimethylaminopyridin.

Die Base wird im allgemeinen in einer Menge von 1 mol bis 4 mol, bevorzugt von 1,2 mol bis 3 mol jeweils bezogen auf 1 mol der Verbindung der Formel (X) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

Die Verbindungen der allgemeinen Formeln (VII), (VIII), (IX) und (X) sind an sich bekannt oder nach üblichen Methoden herstellbar.

Die Verbindungen der allgemeinen Formel (III) können hergestellt werden, indem man Verbindungen der allgemeinen Formel (XI) 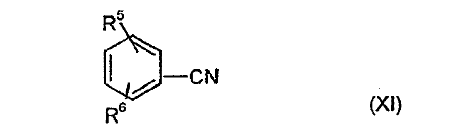 in welcher
R⁵ und R⁶ die oben angegebene Bedeutung haben,
mit Ammoniumchlorid in Toluol und in Anwesenheit von Trimethylaluminium in Hexan in einem Temperaturbereich von -20°C bis Raumtemperatur, vorzugsweise bei 0°C und Normaldruck umsetzt und das entstehende Amidin, gegebenenfalls in situ, mit Hydrazin-hydrat umsetzt.

Die Verbindungen der allgemeinen Formel (XI) sind an sich bekannt oder nach üblichen Methoden herstellbar.

Die Verbindungen der allgemeinen Formel (IV) sind teilweise bekannt oder neu und können dann nach bekannten Methoden [vgl. David R. Marshall, Chemistry and Industry, 2 May 1983, 331-335] hergestellt werden.

Die Verbindungen der allgemeinen Formel (V) sind an sich neu, können aber aus den Verbindungen der allgemeinen Formel (IV) nach der Publikation Organikum, VEB Deutscher Verlag der Wissenschaften, Berlin 1974, Seite 338 - 339, hergestellt werden.

### Ausgangsverbindungen

### Beispiel 1A

### 2-Butyrylaminopropionsäure

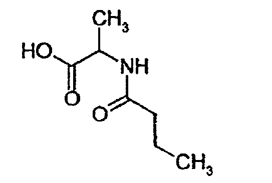

22,27 g (250 mmol) D,L-Alanin und 55,66g (550 mmol) Triethylamin werden in 250 ml Dichlormethan gelöst und die Lösung auf 0°C abgekühlt. 59,75 g (550 mmol) Trimethylsilylchlorid werden zugetropft und die Lösung 1 Stunde bei Raumtemperatur und eine Stunde bei 40°C gerührt. Nach dem Abkühlen auf -10°C werden 26,64 g (250 mmol) Buttersäurechlorid zugetropft und die resultierende Mischung 2 Stunden bei -10°C und eine Stunde bei Raumtemperatur gerührt.

Unter Eiskühlung werden 125 ml Wasser zugetropft und die Reaktionsmischung 15 Minuten bei Raumtemperatur gerührt. Die wäßrige Phase wird bis zur Trockene eingedampft, der Rückstand mit Aceton verrieben und die Mutterlauge abgesaugt. Nach dem Entfernen des Lösungsmittels wird der Rückstand chromatographiert. Das erhaltene Produkt wird in 3N Natronlauge gelöst und die resultierende Lösung bis zur Trockene eingedampft. Es wird mit konz. HCI aufgenommen und wieder bis zur Trockene eingedampft. Es wird mit Aceton verrührt, vom ausgefallenen Feststoff abgesaugt und das Lösungsmittel im Vakuum entfernt. Man erhält 28,2 g (71 %) eines zähen Öls, das nach einiger Zeit kristallisiert.

200 MHz ¹H-NMR (DMSO-d6): 0.84, t, 3H; 1.22, d, 3H; 1.50, hex, 2H; 2.07, t, 2H; 4.20, quin., 1H; 8.09, d,1H.

### Beispiel 3A

### 2-Ethoxybenzonitril

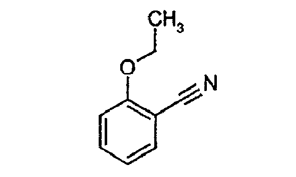

25 g (210 mmol) 2-Hydroxybenzonitril werden mit 87 g Kaliumcarbonat und 34,3 g (314,8 mmol) Ethylbromid in 500 ml Aceton über Nacht refluxiert. Es wird vom Feststoff abfiltriert, das Lösungsmittel im Vakuum entfernt und der Rückstand im Vakuum destilliert. Man erhält 30,0 g (97 %) einer farblosen Flüssigkeit.

200 MHz ¹H-NMR (DMSO-d6): 1.48, t, 3H; 4.15, quart., 2H; 6.99, dt, 2H; 7.51, dt, 2H.

### Beispiel 4A

### 2-Ethoxybenzamidinhydrochlorid

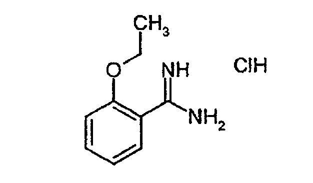

21,4 g (400 mmol) Ammoniumchlorid werden in 375 ml Toluol suspendiert und die Suspension auf 0°C abgekühlt. 200 ml einer 2M Lösung von Trimethylaluminium in Hexan werden zugetropft und die Mischung bis zur beendeten Gasentwicklung bei Raumtemperatur gerührt. Nach Zugabe von 29,44 g (200 mmol) 2-Ethoxybenzonitril wird die Reaktionsmischung über Nacht bei 80°C (Bad) gerührt.

Die abgekühlte Reaktionsmischung wird unter Eiskühlung zu einer Suspension aus 100 g Kieselgel und 950 ml Chloroform gegeben und die Mischung 30 Minuten bei Raumtemperatur gerührt. Es wird abgesaugt und mit der gleichen Menge Methanol nachgewaschen. Die Mutterlauge wird eingedampft, der erhaltene Rückstand mit einer Mischung aus Dichlormethan und Methanol (9:1) verrührt, der Feststoff abgesaugt und die Mutterlauge eingedampft. Man erhält 30,4 g (76 %) farblosen Feststoff.

200 MHz ¹H-NMR (DMSO-d6): 1.36, t, 3H; 4.12, quart., 2H; 7.10, t, 1H; 7.21, d, 1H; 7.52, m, 2H; 9.30, s, breit, 4H.

### Beispiel 10A

### 2-(2-Ethoxy-phenyl)-5-methyl-7-propyl-3H-imidazo[5,1-f][1,2,4]trazin-4-on

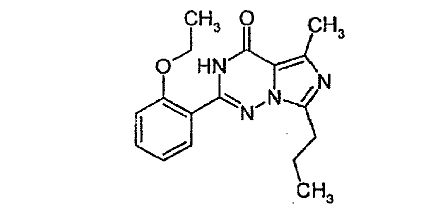

7,16 g (45 mmol) 2-Butyrylamino-propionsäure werden mit 10,67 g Pyridin in 45 ml THF gelöst und nach Zugabe einer Spatelspitze DMAP zum Rückfluß erhitzt. 12,29 g (90 mmol) Oxalsäure-ethylesterchlorid werden langsam zugetropft und die Reaktionsmischung wird 3 Stunden refluxiert. Es wird auf Eiswasser gegossen, dreimal mit Ethylacetat extrahiert, über Natriumsulfat getrocknet und einrotiert. Der Rückstand wird in 15 ml Ethanol aufgenommen und mit 2,15 g Natriumhydrogencarbonat 2,5 Stunden refluxiert. Die abgekühlte Lösung wird filtriert.

Zu einer Lösung von 9,03 g (45 mmol) 2-Ethoxybenzamidinhydrochlorid in 45 ml Ethanol tropft man unter Eiskühlung 2,25 g (45 mmol) Hydrazinhydrat zu und rührt die resultierende Suspension noch 10 Minuten bei Raumtemperatur. Zu dieser Reaktionsmischung gibt man die oben beschriebene ethanolische Lösung und rührt 4 Stunden bei 70°C Badtemperatur. Nach Filtration wird eingedampft, der Rückstand zwischen Dichlormethan und Wasser verteilt, die organische Phase über Natriumsulfat getrocknet und das Lösungsmittel im Vakuum entfernt.

Dieser Rückstand wird in 60 ml 1,2-Dichlorethan gelöst und nach Zugabe von 7,5 ml Phosphoroxychlorid 2 Stunden refluxiert. Es wird mit Dichlormethan verdünnt und durch Zugabe von Natriumhydrogencarbonatlösung und festem Natriumhydrogencarbonat neutralisiert. Die organische Phase wird getrocknet und das Lösungsmittel im Vakuum entfernt. Chromatographie mit Ethylacetat und Kristallisation ergeben 4,00 g (28 %) farblosen Feststoff, R_{f}=0,42 (Dichlormethan/Methanol=95:5)

200 MHz ¹H-NMR (CDCl₃): 1.02, t, 3H; 1.56, t, 3H; 1.89, hex, 2H; 2.67, s, 3H; 3.00, t, 2H; 4.26, quart., 2H; 7.05, m, 2H; 7.50, dt, 1H; 8.17, dd, 1H; 10.00, s, 1H.

### Beispiel 15A

### 4-Ethoxy-3-(5-methyl-4-oxo-7-propyl-3,4-dihydro-imidazo[5,1-f][1,2,4]triazin-2-yl)-benzolsulfonsäurechlorid

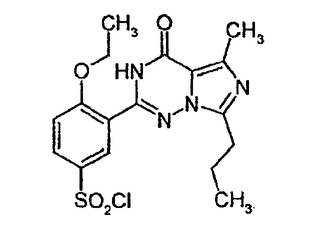

2,00 g (6,4 mmol) 2-(2-Ethoxy-phenyl)-5-methyl-7-propyl-3H-imidazo[5,1-f][1,2,4]-triazin-4-on werden langsam zu 3,83 ml Chlorsulfonsäure bei 0°C gegeben. Die Reaktionsmischung wird bei Raumtemperatur über Nacht gerührt, auf Eiswasser gegossen und mit Dichlormethan extrahiert. Man erhält 2,40 g (91 %) farblosen Schaum.

200 MHz¹H-NMR (CDCl₃): 1.03, t, 3H; 1.61, t, 2H; 1.92, hex, 2H; 2.67, s, 3H; 3.10, t, 2H; 4.42, quart., 2H; 7.27, t, 1H; 8.20, dd, 1H; 8.67, d, 1H; 10.18, s, 1H.

### Beispiel 19A

### (4-Piperidinylmethyl)-phosphonsäurediethylester

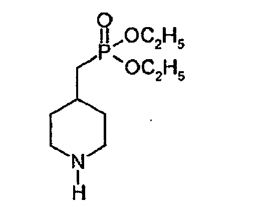

Man legt 2,11 g (528 mmol) 60%iges Natriumhydrid in 50 ml absolutem Tetrahydrofuran vor und tropft 15,7 g (52,8 mmol) Methandiphosphonsäurediethylester hinzu. Man rührt noch 30 Minuten bei Raumtemperatur und tropft dann 10,1 g (52,8 mmol) 1-Benzyl-4-piperidon hinzu. Man rührt eine Stunde bei Raumtemperatur und eine Stunde unter Rückfluß, engt ein, versetzt mit Wasser, extrahiert dreimal mit Dichlormethan, trocknet über Natriumsulfat und engt ein. Der Rückstand wird in 50 ml Ethanol an 1,7 g 10%iger Palladium-Aktivkohle bei Raumtemperatur und 3 bar hydriert. Man saugt den Katalysator ab und engt das Filtrat ein.
Ausbeute: 12,5 g (100% d.Th.)

400 MHz, ¹H-NMR (CDCl₃): 1,13, m, 2H; 1,32, t, 6H; 1,69, dd, 2H; 1,74 - 1,95, m, 4H; 2,62, dt, 2H; 3,05, m, 2H; 4,1, m, 4H.

### Beispiel 20A

### 5-Methyl-4-furoxancarbaldehyd

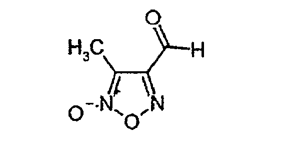

40 g (571 mmol) Crotonaldehyd werden in 80 ml Essigsäure gelöst und bei 0°C mit einer Lösung von 137 g (1,99 mol) Natriumnitrit in 300 ml Wasser tropfenweise versetzt. Man rührt 2 Stunden bei Raumtemperatur. Es wird mit 800 ml Wasser verdünnt und 3 mal mit Dichlormethan extrahiert. Nach Trocknen der organischen Phase erhält man durch Chromatographie (Cyclohexan/Ethylacetat) 13,8 g (18,9 %) 5-Methyl-4-furoxancarbaldehyd.

200 MHz ¹H-NMR(CDCl₃):2.39, s, 3H; 10.10, s, 1H.

### Beispiel 21A

### 5-Methyl-4-furoxancarbonsäurechlorid

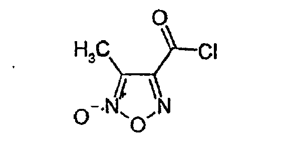

13,5 g (105 mmol) 5-Methyl-4-furoxancarbaldehyd werden in 200 ml Aceton gelöst und bei 0°C tropfenweise mit einer Lösung von 16,86 g (168 mmol) Chromtrioxid in 120 ml einer 2.2M Schwefelsäure versetzt. Man rührt 2 Stunden bei 10-15°C und bei Raumtemperatur über Nacht. Unter Kühlung werden 100 ml Isopropanol zugetropft und nach 30 Minuten das Lösungsmittel im Vakuum entfernt. Die wäßrige Phase wird 3 mal mit Ether extrahiert, die organische Phase über Magnesiumsulfat getrocknet und das Lösungsmittel im Vakuum entfernt. Der Rückstand wird in 1M Natriumhydroxidlösung gelöst und die Lösung 3 mal mit Ether extrahiert. Die wäßrige Phase wird sauer gestellt und 3 mal mit Ether extrahiert. Die organische Phase wird getrocknet und das Lösungmittel im Vakuum entfernt. Der Rückstand wird mit Petrolether verrührt und abgesaugt.

6,92 g des Rückstandes werden mit 10ml Thionylchlorid in 20 ml Dichlormethan 6 Stunden refluxiert. Es wird mit Toluol verdünnt, filtriert und einrotiert. Der Rückstand wird wiederum in Dichlormethan aufgenommen, mit 10 ml Thionylchlorid versetzt und 48 Stunden refluxiert. Das Lösungsmittel wird im Vakuum entfernt und der Rückstand im Vakuum destilliert. Man erhält 2,00 g (25 %) farblose Kristalle.

200 MHz ¹H-NMR (CDCl₃): 2.41, s.

### Beispiel 22A

### 1-(5-Methyl-4-furexancarbonyl)-4-tert-butyl-oxycarbonyl-piperazin

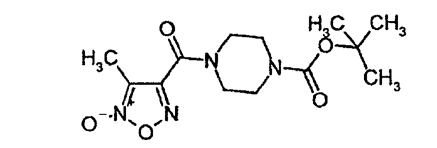

2,75 g (14,7 mmol) Boc-Piperazin werden mit 1,49 g Triethylamin in 20 ml Dichlormethan gelöst und bei 0°C portionsweise mit 2,00 g (12,3 mmol) 5-Methyl-4-furoxancarbonsäurechlorid versetzt. Es wird 30 Minuten bei 0°C und 2 Stunden bei Raumtemperatur gerührt, mit Dichlormethan verdünnt und mit Wasser gewaschen. Das Lösungsmittel wird im Vakuum entfernt und der Rückstand durch Chromatographie (Cyclohexan/Ethylacetat) gereinigt. Man erhält 3,33 g (87 %) 1-(5-Methyl-4-furoxancarbonyl)-4-tert-butyl-oxycarbonyl-piperazin.

200 MHz ¹H-NMR (CDCl₃): 1.50, s, 9H; 2.30, s, 3H; 3.55, m, 4H; 3.78, m, 2H; 3.87, m, 2H.

### Beispiel 23A

### 1-(5-Methyl-4-furoxancarbonyl)-piperazin Trifluoracetat

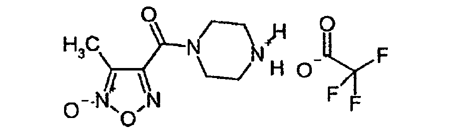

3,12 g (10 mmol) 1-(5-Methyl-4-furoxancarbonyl)-4-tert-butyl-oxycarbonyl-piperazin werden in 20 ml Dichlormethan gelöst und bei 0°C mit 2 ml Trifluoressigsäure versetzt. Man läßt auf Raumtemperatur aufwärmen und rührt 72 Stunden. Nach Zugabe von 10 ml Ether wird der Niederschlag abgesaugt und getrocknet. Man erhält 2,47 g (83 %)1-(5-Methyl-4-furoxancarbonyl)-piperazin Trifluoracetat.

200 MHz ¹H-NMR (DMSO-d₆): 2.18, s, 3H; 3.18, m, 2H; 3.25, m, 2H; 3.83, m, 2H; 3.90, m, 2H; 8.89, s, breit, 2H.

## Patentansprüche

1. Verbindungen der Formel (V) 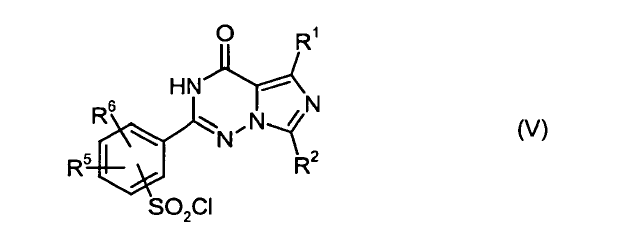 in welcher
R¹ für Methyl, R² für Propyl, R⁵ für Wasserstoff und R⁶ in 1-Position für Ethoxy steht und die SO₂Cl Gruppe in 4-Position steht.

2. Verbindung der Formel (IV) 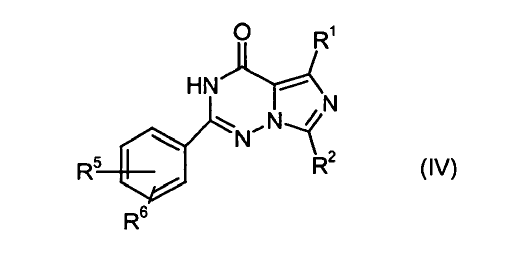 in welcher
R¹ für Methyl, R² für Propyl, R⁵ für Wasserstoff und R⁶ in 1-Position für Ethoxy steht.

3. Verwendung von Verbindungen wie in den Ansprüchen 1 und 2 definiert zur Herstellung von Verbindungen der Formel

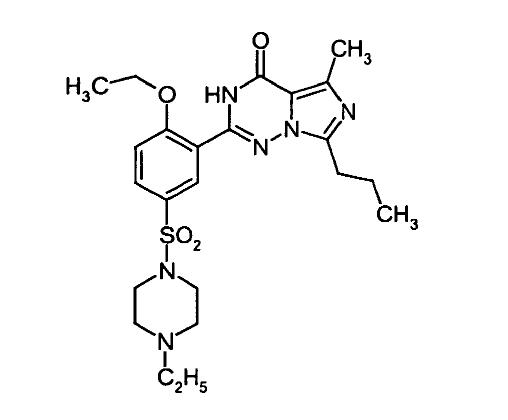

4. Verfahren zur Herstellung der in Anspruch 2 definierten Verbindungen der Formel (IV), **dadurch gekennzeichnet, daß** man
Verbindungen der allgemeinen Formel (II) 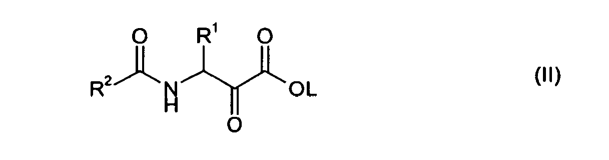 in welcher
R¹ und R² die in Anspruch 2 angegebenen Bedeutungen haben
und
L für geradkettiges oder verzweigtes Alkyl mit bis zu 4 Kohlenstoffatomen steht,
mit Verbindungen der allgemeinen Formel (III) 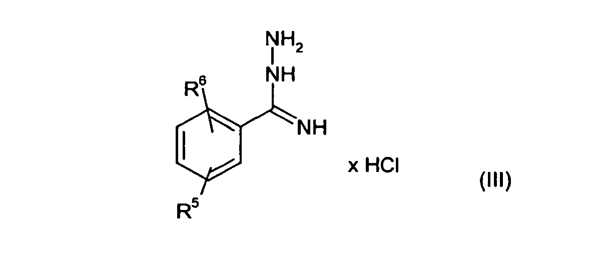 in welcher
R⁵ und R⁶ die in Anspruch 2 angegebenen Bedeutungen haben,
in dem System Ethanol und Phosphoroxytrichlorid / Dichlorethan umsetzt.

5. Verfahren zur Herstellung der in Anspruch 1 definierten Verbindungen der Formel (V), **dadurch gekennzeichnet, daß** man
Verbindungen der Formel (IV) 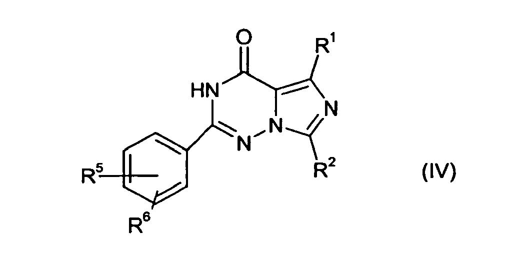 in welcher
R¹, R², R⁵ und R⁶ die in Anspruch 1 angegebenen Bedeutungen haben, mit Chlorsulfonsäure umsetzt.

## Claims

1. Compounds of the formula (V) 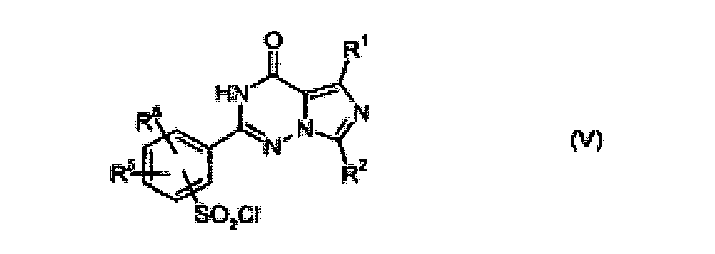 in which
R¹ represents methyl, R² represents propyl, R⁵ represents hydrogen and R⁶ in the 1-position represents ethoxy and the SO₂Cl group is located in the 4-position.

2. Compound of the formula (IV) 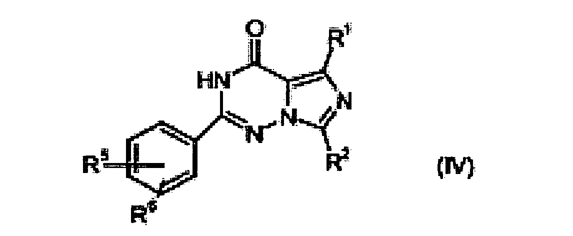 in which R¹ represents methyl, R² represents propyl, R⁵ represents hydrogen and R⁶ in the 1-position represents ethoxy.

3. Use of compounds as defined in Claims 1 and 2 for preparing compounds of the formula

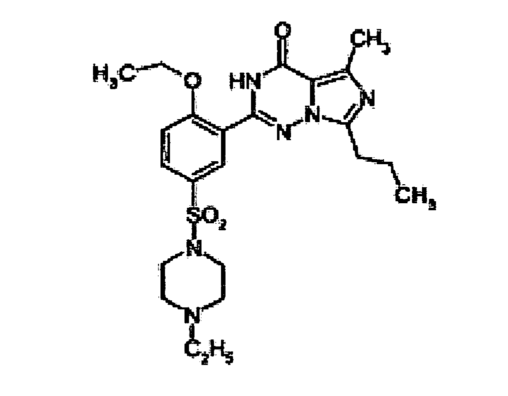

4. Process for preparing the compounds of the formula (IV) defined in Claim 2, **characterized in that** compounds of the general formula (II) 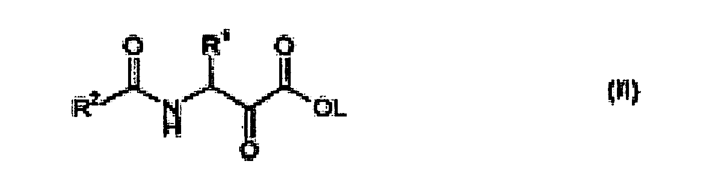 in which
R¹ and R² have the meanings given in Claim 2
and
L represents straight-chain or branched alkyl having up to 4 carbon atoms
are reacted with compounds of the general formula (III) 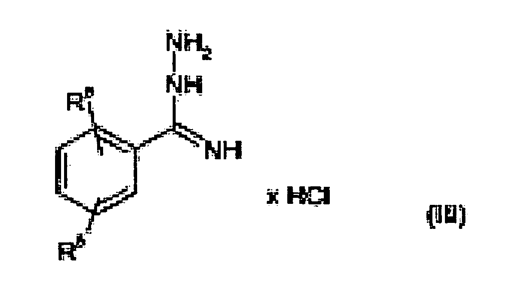 in which
R⁵ and R⁶ have the meanings given in Claim 2
in the system ethanol and phosphorus oxytrichloride/dichloroethane.

5. Process for preparing the compounds of the formula (V) defined in Claim 1, **characterized in that**
compounds of the formula (IV) 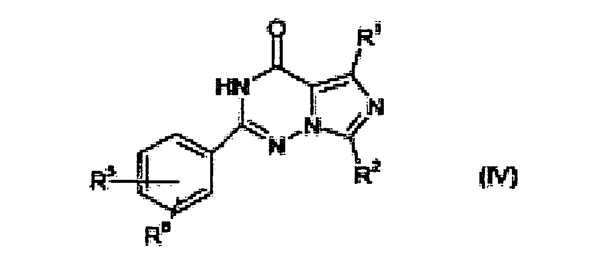 in which
R¹, R², R⁵ and R⁶ have the meanings given in Claim 1 are reacted with chlorosulfonic acid.

## Revendications

1. Composés de formule (V) 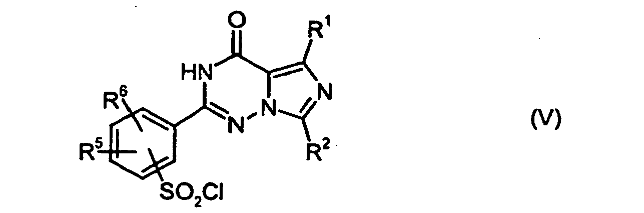 dans laquelle
R¹ représente méthyle, R² représente propyle, R⁵ représente hydrogène et R⁶ en position 1 représente éthoxy et le groupe SO₂Cl se trouve en position 4.

2. Composé de formule (IV) 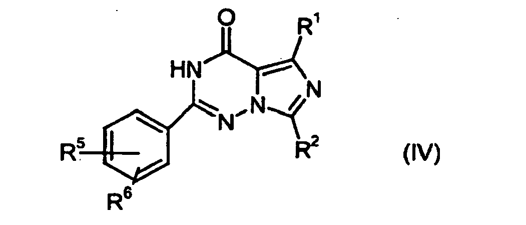 dans laquelle
R¹ représente méthyle, R² représente propyle,R⁵ représente hydrogène et R⁶ en position 1 représente éthoxy.

3. Utilisation de composés tels que définis dans les revendications 1 et 2 pour la préparation de composés de formule

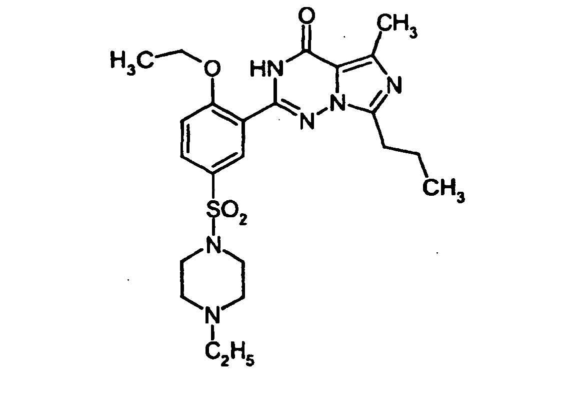

4. Procédé pour la préparation des composés définis dans la revendication 2 de formule (IV), **caractérisé en ce qu'**on transforme des composés de formule générale (II) 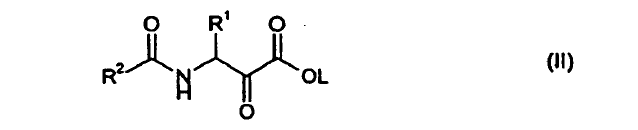 dans laquelle
R¹ et R² présentent les significations indiquées dans la revendication 2 et
L représente alkyle linéaire ou ramifié comprenant jusqu'à 4 atomes de carbone,
avec des composés de formule générale (III) 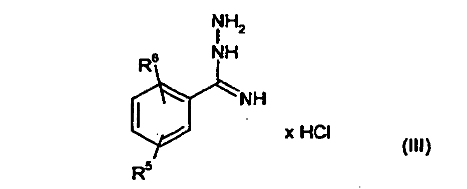 dans laquelle
R⁵ et R⁶ présentent les significations indiquées dans la revendication 2, dans le système éthanol et phosphoroxytrichlorure/dichloroéthane.

5. Procédé pour la préparation des composés définis dans la revendication 1 de formule (V), **caractérisé en ce qu'**on transforme des composés de formule (IV) 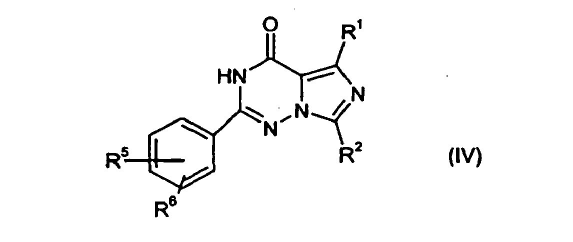 dans laquelle
R¹, R², R⁵ et R⁶ présentent les significations indiquées dans la revendication 1, avec de l'acide chlorosulfonique.
